(19) Europäisches Patentamt
European Patent Office
Office européen des brevets

(11) Publication number: 0 104 596 A2

# (12) EUROPEAN PATENT APPLICATION

(21) Application number: 83109362.0

(22) Date of filing: 20.09.83

(51) Int. Cl.³: C 07 D 491/048
//A61K31/535, (C07D491/048, 307/00, 209/00)

(30) Priority: 25.09.82 JP 167307/82
17.05.83 JP 85101/83

(43) Date of publication of application: 04.04.84 Bulletin 84/14

(84) Designated Contracting States: DE FR GB IT

(71) Applicant: TAISHO PHARMACEUTICAL CO. LTD
24-1 Takata 3-chome Toshima-ku
Tokyo 171(JP)

(72) Inventor: Nakashima, Yoshimoto
18-16, Goban-cho
Ageo Saitama, 362(JP)

(72) Inventor: Kawashima, Yutaka
1731-1, Akoda
Tatebayashi Gunma, 374(JP)

(72) Inventor: Satoh, Masakazu
Maruei-so 3; 25-1 Nara-cho
Ohmiya Saitama, 330(JP)

(72) Inventor: Sota, Kaoru
1158-11, Shimotomi
Tokorozawa Saitama, 359(JP)

(74) Representative: Schön, Alfred, Dr. et al,
Patentanwälte Müller-Boré, Deufel, Schön, Hertel
Lewald, Otto Isartorplatz 6 Postfach 26 02 47
D-8000 München 26(DE)

## (54) Furoindole derivatives.

(57) Novel furoindole derivatives of the formula

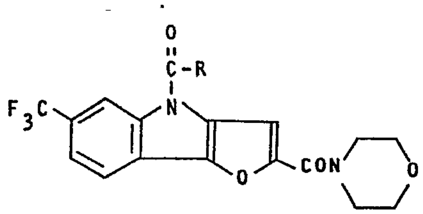

wherein R is isobutyl, 3-heptyl or isopropoxy, are useful as analgesic and anti-inflammatory agents.

EP 0 104 596 A2

# FUROINDOLE DERIVATIVES

This invention relates to novel furoindole derivatives which have analgesic, anti-inflammatory and antipyretic activity.

Although being not specifically disclosed in the past, 2-morpholinocarbonyl-6-trifluoromethyl-4H-furo[3,2-b]indole is within a generic teachings of the Japanese Patent Application Laid-open No. 51-63194. Compounds having alkylcarbonyl or alkoxycarbonyl substituents at the 4-position of the furo[3,2-b]indole nucleus are not known.

The compounds of the present invention are compounds represented by the general formula

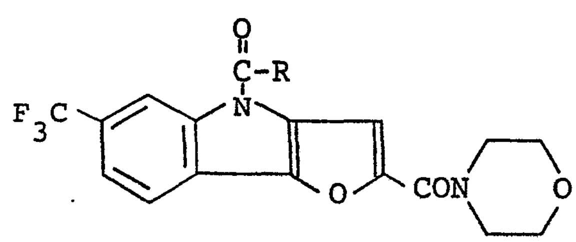

wherein R is isobutyl, 3-heptyl or isopropoxy.

The object of the present invention is to provide novel furoindole derivatives possessing excellent analgesic activity.

The compounds of the present invention are prepared, for example, by the following methods.

Ethyl 6-trifluoromethyl-4H-furo[3,2-b]indole-2-carboxylate is converted to 6-trifluoromethyl-4H-furo[3,2-b]indole-2-carbonyl chloride in accordance with the method

described in the Japanese Patent Application Laid-Open No. 52-3096. This compound is condensed with morpholine in a solvent such as benzene, acetone, chloroform, dichloromethane or the like to give 2-morpholinocarbonyl-6-trifluoromethyl-4H-furo[3,2-b]-indole. This reaction may be carried out with stirring at room temperature for several hours.

2-morpholinocarbonyl-6-trifluoromethyl-4H-furo[3,2-b]-indole thus obtained is reacted with a compound of the formula

R-CO-X

wherein X is halogen and R is defined above, in the presence of an alkali such as sodium hydride, sodium methoxide or the like in an organic solvent such as dimethylformamide, ethyl ether, acetone or the like with stirring for 20 minutes to several hours to give the compound of formula I.

The compounds of the present invention have excellent analgesic, anti-inflammatory and antipyretic activity when compared with the known furoindole compounds, therefore they can be used as medicaments to mammals. For these purposess, they may be administered orally in a conventional dosage form such as tablet, capsule or powder prepared according to conventional pharmaceutical practices. A single dose, or preferably, 2 to 4 divided daily doses, provided on a basis of about 0.1 to 5 mg/kg/day, is appropriate.

When each of 10 male rats of Wistar strain weighing 100 - 200 g was administered orally with 2000 mg/kg of the compound of formula I wherein R is isobutyl, and with 1000 mg/kg of the compound of formula I wherein R is 3-heptyl or

isopropoxy, respectively, no death was observed.

The following tests are illustrative of procedures of the biological assay for the compounds of the present invention, and the results are shown in Table 1.

Test 1. Analgesic Effect

(1) Acetic Acid Writhing Method [Koster, Anderson and De Beer: *Fed. Proc.*, *18*, 412(1959)]

10 male ddY mice weighing 19 - 23 g were used in testing each compound. The test compounds were administered orally 30 minutes before in the intraperitoneal injection (0.1 ml/ 10 g ) of 0.7 % acetic acid. The number of writhes were counted in each mouse during a period spanning 10 to 20 minutes after the acetic acid injection.

The percent inhibition was calculated as compared with the number of writhes in the control group, and the $ED_{50}$ values were calculated from the inhibitory percent.

(2) Tail Pressure Method [Takagi and Kameyama: *Yakugaku Zasshi*, *78*, 553(1958)]

10 male ddY mice weighing 20 - 25 g were used in testing each compound. The pressure stimulus was applied to the base of the mouse tail. The test compounds were administered orally and the pain threshold was measured every 30 minutes for 120 minutes.

Animals of which post-drug pain threshold raised more than 50 % as compared with its pre-drug threshold were regarded as positive for the analgesic activity, and the $ED_{50}$ values were calculated from the positive percent.

(3) Randall & Selitto Method [Randall and Selitto: *Arch. Int. Pharmacodyn. 111*, 409(1957)]

6 male Wistar rats weighing 90 - 110 g were used in testing each compound. The pain threshold was measured with an analgesic meter (Ugo Basile). The test compounds were adminstered orally 2 hours after the subplantar injection(0.1 ml/rat) of 20 % brewer's yeast suspension into the right hind paw, and the pain threshold was measured every 1 hour for 4 hours.

Animals of which post-drug pain threshold raised more than 50 % as compared with the threshold in control group were regarded as positive for the analgesic activity, and the $ED_{50}$ values were calculated from the positive percent.

(4) Adjuvant-induced Arthritic Pain [Kuzuna and Kawai: *Chem. Pharm. Bull., 23*, 1184(1975)]

6 male S.D. rats weighing 160 - 200 g were used in testing each compound. Polyarthritis was induced by the intradermal injection (0.1 ml/rat) of 0.5 % Mycobacterium butyricum suspension into the distal portion of the tail. The pain stimulus was applied by a gentle flection of the right tarso-tibial joints repeatedly 5 times at intervals 4 to 5 seconds, the vocalization following the pain stimulus was regarded as pain response. 15 to 20 days later rats that showed the pain response were selected and the test compounds were administered orally, and the pain response was measured every 1 hour for 5 hours.

Animals that did not show the pain response were



BAD ORIGINAL

regareded as positive for the analgesic activity, and the $ED_{50}$ values were calculated from the inhibitory percent.

Table I

| Compound of Formula I | Analgesic action ($ED_{50}$) mg/kg | | | |
|---|---|---|---|---|
| | Acetic acid writhing method | Tail pressure method | Randall & Selitto method | Adjuvant-induced arthritic pain method |
| R: 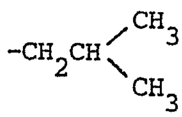 | 8.0 | 68.3 | 11.8 | 19.4 |
| R: 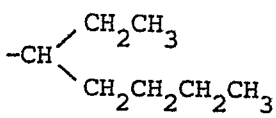 | 0.8 | 7.0 | 1.7 | 1.7 |
| R: 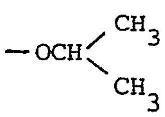 | 3.7 | 27.8 | 5.0 | 5.5 |
| R: -H | >100 | >100 | >100 | >100 |

The following examples are illustrative of the present invention.

Example 1

To a mixture of 20 ml of a 10 % aqueous sodium

hydroxide solution and 50 ml of acetone was added 5 g of ethyl 6-trifluoromethyl-4H-furo[3,2-b]indole-2-carboxylate (m.p. 224 -225 °C), followed by stirring at room temperature for 30 minutes. The mixture was concentrated to evaporate the acetone, and acidified with hydrochloric acid. Filtration of the precipitate which formed gave 4.2 g of 6-trifluoromethyl-4H-furo[3,2-b]indole-2-carboxylic acid, which was then dried thoroughly, and 50 ml of benzene was added. To the mixture was poured 5 ml of thionyl chloride, followed by reflux for one hour. An excess thionyl chloride and benzene were evaporated to give 3.5 g of 6-trifluoromethyl-4H-furo[3,2-b]-indole-2-carbonyl chloride, which was then dissolved in a mixture of 10 ml of acetone and 10 ml of dichloromethane. To the solution was added dropwise a solution of 3 g of morpholine in 5 ml of dichloromethane with stirring. After completion of addition, stirring was continued at room temperature for 30 minutes. Water was added to the resulting mixture, and the crystals which formed were collected by filtration, washed with water, dried and recrystallized from a mixture of acetone and petroleum ether to give 3.0 g of 2-morpholinocarbonyl-6-trifluoromethyl-4H-furo[3,2-b]indole (m.p. 221.5 - 222.5 °C).

A solution of the resulting crystals in 40 ml of dimethylformamide was added dropwise to a suspension of 0.23 g of sodium hydride in 5 ml of dimethylformamide with stirring. To the mixture was added dropwise 1.6 g of 2-ethylhexanoyl chloride, followed by stirring at room temperature for 1 hour.

100 ml of dichloromethane was added to the reaction mixture, the mixture was washed with dilute hydrochloric acid and saturated salt solution, dried with anhydrous sodium sulfate. Dichloromethane was evaporated, and the residue was recrystallized from n-hexane to give 2.3 g of 4-(2-ethylhexanoyl)-2-morpholinocarbonyl-6-trifluoromethyl-furo-[3,2-b]-indole.

m.p. 107 - 108 °C.

Elementary analysis for $C_{24}F_3H_{27}N_2O_4$

Calcd. (%): C 62.05, H 5.87, N 6.03

Found (%): C 62.18, H 5.82, N 5.95

Mass: m/e ($M^+$) 464

Example 2

To a mixture of 100 ml of 10 % aqueous solution of sodium hydroxide and 100 ml of acetone was added 18 g of ethyl 6-trifluoromethyl-4H-furo[3,2-b]indole-2-carboxylate, followed by stirring at room temperature for 1 hour. The mixture was concentrated to evaporate the acetone and acidified with hydrochloric acid. Filtration of the precipitate gave 16 g of 6-trifluoromethyl-4H-furo[3,2-b]indole-2-carboxylic acid. To this were added 20 ml of thionyl chloride and 50 ml of benzene, and the mixture was heated under reflux for one hour. An excess thionyl chloride and benzene were evaporated, and there was obtained 16 g of 6-trifluoromethyl-4H-furo[3,2-b]-indole-2-carbonyl chloride. This was dissolved in a mixture

of 20 ml of acetone and 20 ml of dichloromethane, and added dropwise to a solution of 10 g of morpholine in 20 ml of dichloromethane with stirring. After completion of the addition, the mixture was stirred at room temperature for 30 minutes. The crystals which formed were then collected by filtration, washed with water and dried to give 15 g of 2-morpholinocarbonyl-6-trifluoromethyl-4H-furo[3,2-b]indole. This was dissolved in 100 ml of dimethylformamide, and the solution was added dropwise to a suspension of 1.1 g of sodium hydride in 10 ml of dimethylformamide with stirring. To the solution was added dropwise 8 g of isopropyl chloroformate, and the mixture was stirred at room temperature for one hour. Finally, the reaction mixture was poured into water to precipitate crystals, which were then recrystallized from acetone to give 15 g of 4-isopropoxycarbonyl-2-morpholinocarbonyl-6-trifluoromethyl-furo-[3,2-b]indole.

m.p. 113 - 115 °C

Elementary analysis for $C_{20}F_3H_{19}N_2O_5$

Calcd. (%): C 56.60, H 4.51, N 6.60

Found (%): C 56.51, H 4.57, N 6.48

Mass: m/e ($M^+$) 424

Example 3

Following the procedure similar to that of Example 2 and using 25 g of ethyl 6-trifluoromethyl-4H-furo[3,2-b]-indole-2-carboxylate in place of 18 g of the same and 8 g of isovaleryl chloride in place of 8 g of isopropyl

chloroformate, there was obtained 21 g of 4-isobutylcarbonyl-2-morpholinocarbonyl-6-trifluoromethyl-furo[3,2-b]indole.

m.p. 146 - 147 °C

Elementary analysis for $C_{21}F_3H_{21}N_2O_4$

Calcd. (%): C 59.71, H 5.01, N 6.63

Found (%): C 59.45, H 4.98, N 6.60

Mass: m/e ($M^+$) 422

What is claimed is :

1. A furoindole derivative of the formula

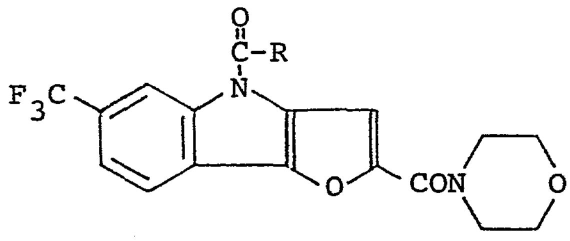

wherein R is isobutyl, 3-heptyl or isopropoxy.

2. 4-isobutylcarbonyl-2-morpholinocarbonyl-6-trifluoromethyl-furo[3,2-b]indole.

3. 4-(2-ethylhexanoyl)-2-morpholinocarbonyl-6-trifluoromethyl-furo[3,2-b]indole.

4. 4-isopropoxycarbonyl-2-morpholinocarbonyl-6-trifluoromethyl-furo[3,2-b]indole.